(19) Europäisches Patentamt / European Patent Office / Office européen des brevets

(11) **EP 2 823 830 A1**

(12) **EUROPEAN PATENT APPLICATION**
published in accordance with Art. 153(4) EPC

(43) Date of publication:
**14.01.2015 Bulletin 2015/03**

(21) Application number: **13757451.3**

(22) Date of filing: **08.03.2013**

(51) Int Cl.:
*A61L 31/00* (2006.01)        *A61K 9/06* (2006.01)
*A61K 9/08* (2006.01)        *A61K 38/00* (2006.01)
*A61K 47/14* (2006.01)        *A61K 47/22* (2006.01)
*A61K 47/26* (2006.01)        *A61K 47/36* (2006.01)
*A61K 47/42* (2006.01)        *A61P 7/04* (2006.01)
*A61P 17/02* (2006.01)        *A61P 41/00* (2006.01)
*A61P 43/00* (2006.01)

(86) International application number:
**PCT/JP2013/056472**

(87) International publication number:
**WO 2013/133413 (12.09.2013 Gazette 2013/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **09.03.2012 JP 2012052867**

(71) Applicant: 3-D Matrix, Ltd.
**Chiyoda-ku
Tokyo 102-0083 (JP)**

(72) Inventors:
• **TAKAMURA, Kentaro
Tokyo 102-0083 (JP)**
• **KOBAYASHI, Satoru
Tokyo 102-0083 (JP)**
• **MATSUDA, Noriaki
Tokyo 102-0083 (JP)**

(74) Representative: **Hoffmann Eitle
Patent- und Rechtsanwälte PartmbB
Arabellastraße 30
81925 München (DE)**

(54) **MUCOSA-ELEVATING AGENT**

(57) Provided is a mucosa-elevating agent containing 0.1% to 1.0% of a peptide, wherein the peptide is an amphipathic protein having 8 to 200 amino acid residues in which hydrophilic amino acids and hydrophobic amino acids are alternately bonded, and is a self-assembling peptide that exhibits a β structure in an aqueous solution at physiological pH and/or in the presence of cations.

EP 2 823 830 A1

**Description**

TECHNICAL FIELD

The present invention relates to a mucosa-elevating agent that contains a self-assembling peptide hydrogel.

BACKGROUND ART

Endoscopic mucosal resection (EMR) and endoscopic submucosal dissection (ESD), which enable lowly invasive removal of polyps, cancerous lesions and the like from the digestive tract, have become the first choice of surgery accompanying progress made in the field of endoscopic technology.

Endoscopic surgery is a procedure used to remove lesions without laparotomy, and consists of injecting hypertonic saline or high molecular weight polymer solution into the submucosal layer to distend and elevate the mucosa followed by resecting or dissecting with a high-frequency therapeutic apparatus.

Since the surgical indications for ESD have expanded in recent years and ESD allows the submucosal layer to be dissected over a wider range than EMR, it is necessary to maintain distension and elevation of the submucosal layer at a sufficient height until dissection is completed due to the high possibility for puncture caused by incising individual muscle fascia.

Although a high-frequency therapeutic apparatus such as an electric scalpel is used to resect and dissect lesions in the case of EMR or ESD, it is necessary to avoid the mucosa-elevating agent having a detrimental effect on the electrical effects and operability thereof.

An example of an existing mucosa-elevating agent is sodium hyaluronate. Although sodium hyaluronate is frequently used in the clinical setting as an effective mucosa-elevating agent, it is associated with shortcomings such as 1) the risk of infection due to being a product of biological origin, and 2) its inability to be filled into a syringe.

Self-assembling peptides have the property of forming a self-aggregate consisting of a large number of peptide molecules arranged in an orderly manner according to the amino acid sequence thereof. Self-assembling peptides have recently attracted attention as a novel material based on their physical, chemical and biological properties.

Self-assembling peptides have a structure in which electrically charged hydrophilic amino acids and electrically neutral hydrophobic amino acids are alternately arranged resulting in an alternating distribution of positive and negative charge, and adopt a $\beta$ structure at physiological pH and salt concentration.

Acidic amino acids selected from among aspartic acid and glutamic acid as well as basic amino acids selected from among arginine, lysine, histidine and ornithine can be used as hydrophilic amino acids. Amino acids that can be used as hydrophobic amino acids consist of alanine, valine, leucine, isoleucine, methionine, phenylalanine, tyrosine, tryptophan, serine, threonine and glycine.

The aforementioned peptide self-assembly occurs under the conditions indicated below.

(1) Electrically charged hydrophilic amino acids and electrically neutral amino acids are unevenly distributed on two sides of the peptide molecule as a result of the peptide molecule adopting a $\beta$ structure in aqueous solution.
(2) Charge is distributed complementarily between adjacent molecules when a $\beta$ structure has been adopted.
(3) Hydrophobic bonds are adequately formed between adjacent molecules when a $\beta$ structure has been adopted.
(4) The charge of amino acid side chains is screened with a monovalent inorganic salt.
(5) Molecules become electrostatically neutral near the isoelectric point of the peptide.

Self-assembly is thought to proceed by the mechanism indicated below when the aforementioned conditions have been satisfied.

(1) Peptide molecules are mutually attracted and approach each other due to the positive charge and negative charge of alternately distributed peptide molecules.
(2) Hydrophobic bonds are formed between the side chains of neutral amino acids of adjacent molecules.
(3) The relative arrangement of adjacent molecules is organized according to the distribution of positive and negative charge, and bonding strength between molecules increases.
(4) Aggregates of molecules gradually become elongated resulting in the formation of nanofibers.

Nanofibers are ultrafine fibers having a thickness of about 10 nm to 20 nm, and have been reported to aggregate in the form of a network and exhibit the form of a gel macroscopically.

The fiber size or pore size and the like of the network structure of the gel is extremely similar to that of a naturally-occurring extracellular matrix (ECM), and research has been conducted on its use as a scaffold for cell culturing.

This peptide hydrogel is biodegradable, and since the decomposition products thereof do not have a detrimental effect on tissue and demonstrate a high degree of bioabsorptivity, it is suitable for cell growth and proliferation.

Since self-assembling peptides are synthesized chemically by solid-phase synthesis and are free of concerns over animal-derived infections, they are attracting further attention as an alternative to sodium hyaluronate or collagen and the like based on recent growing concerns over bovine spongiform encephalopathy (BSE), animal-borne viruses and unknown infectious diseases.

Although the application of a self-assembling peptide to a mucosa-elevating agent is indicated in Patent Document 1, effects for maintaining mucosa elevation and endoscopic elevating effects are not reported in a mucosa elevation experiment conducted on the urinary bladder mucosa of dogs cited in an example. Thus, the mucosa-elevating effects of self-assembling peptide mucosa-elevating agents require further improvement in order to attain the level of clinical application.

PRIOR ART DOCUMENTS

Patent Documents

Patent Document 1: International Publication No. WO 2010/041636

DISCLOSURE OF THE INVENTION

Problems to be Solved by the Invention

An object of the present invention is to provide a self-assembling peptide mucosa-elevating agent that is able to maintain elevation and distension of gastrointestinal mucosa during endoscopy in large mammals, including humans, for an adequate amount of time clinically and is free of concerns over viral and other infectious diseases, and a method for using that mucosa-elevating agent.

Means for Solving the Problems

The inventors of the present invention found that mucosa-elevating effects equal to or greater than those of existing mucosa-elevating agents are demonstrated as a result of applying a self-assembling peptide hydrogel used as a scaffold for cell culturing to mucosa elevation, thereby leading to completion of the present invention. In addition, when an aqueous peptide solution having a concentration of 3% reported in Patent Document 1 is injected into a submucosal layer, although the effect of elevating mucosa is obtained, since the peptide solution per se is highly viscous, it is difficult to inject. In addition, there are also other problems such as difficulty in re-injecting a gel formed by self-assembly of a peptide solution having a concentration of 3% due to the hardness thereof, obstruction of tissue resection during mucosal resection and dissection with an electrical scalpel or other high-frequency therapeutic apparatus, and obstruction of field of view due to adherence of gel to the endoscope. Therefore, as a result of conducting extensive research, the inventors of the present invention found that, in addition to problems observed when using an aqueous peptide solution having a concentration of 3% being no longer encountered, adequate effects for maintaining mucosa elevation are observed, thereby allowing the obtaining of resection and dissection effects, and leading to completion of the present invention.

Namely, the present invention is as described below.

[1] A mucosa-elevating agent containing 0.1% to 1.0% of a peptide, wherein the peptide is an amphipathic protein having 8 to 200 amino acid residues in which hydrophilic amino acids and hydrophobic amino acids are alternately bonded, and is a self-assembling peptide that exhibits a β structure in an aqueous solution at physiological pH and/or in the presence of cations.

[2] The mucosa-elevating agent described in [1], wherein the peptide has a repetitive sequence of a sequence consisting of arginine, alanine, aspartic acid and alanine, a sequence consisting of isoleucine, glutamic acid, isoleucine and lysine, or a sequence consisting of lysine, leucine, aspartic acid and leucine.

[3] The mucosa-elevating agent described in [1] or [2], wherein the peptide is consisted of the amino acid sequence described in SEQ ID NO: 1, SEQ ID NO: 2 or SEQ ID NO: 3.

[4] The mucosa-elevating agent described in any of [1] to [3], further containing a pharmaceutical agent.

[5] The mucosa-elevating agent described in [4], wherein the pharmaceutical agent is a pharmaceutically acceptable pigment.

[6] The mucosa-elevating agent described in [4], wherein the pharmaceutical agent is selected from the group consisting of glucose, sucrose, refined sucrose, lactose, maltose, trehalose, dextran, iodine, lysozyme chloride, dimethyl isopropylazulene, tretinoin tocoferil, iodopovidone, alprostadil alfadex, anisyl alcohol, isoamyl salicylate, $\alpha,\alpha$-dimethylphenylethyl alcohol, bacdanol, helional, silver sulfadiazine, bucladesine sodium, alprostadil alfadex, gentamycin sulfate, tetracycline hydrochloride, fusidate sodium, mupirocin calcium hydrate and isoamyl benzoate.

[7] The mucosa-elevating agent described in any of [1] to [6], which is injected between a mucous membrane and a muscle layer.

[8] The mucosa-elevating agent described in any of [1] to [6], wherein the mucous membrane is gastrointestinal mucosa.

[9] The mucosa-elevating agent described in any of [1] to [6], which is used in mucosal resection.

[10] The mucosa-elevating agent described in any of [1] to [6], which is used in submucosal layer dissection.

[11] The mucosa-elevating agent described in any of [1] to [6], which can be additionally injected.

[12] The mucosa-elevating agent described in any of [1] to [6], which is in a form of being filled in a syringe.

[13] The mucosa-elevating agent described in any of [1] to [6], which has a wound-healing effect.

[14] The mucosa-elevating agent described in any of [1] to [6], which has a scar-preventing effect or constriction-preventing effect.

[15] The mucosa-elevating agent described in any of [1] to [6], which has a hemostatic effect.

[16] The mucosa-elevating agent described in any of [1] to [6], which is in a liquid form that turns into a gel in the body.

The aforementioned pigment is a pharmaceutically acceptable pigment, and is preferably selected from among indigo carmine, brilliant blue FCF, fast green FCF and indocyanine green.

The present invention also relates to an injection preparation for injection into a submucosal layer for elevating a site of resection or dissection during EMR or ESD, or a method for resecting mucosal tissue that has been elevated by injection of a liquid into a submucosal layer.

Effects of the Invention

In addition to the main component thereof in the form of a self-assembling peptide fulfilling the role of a mucosa-elevating agent, the tissue occluding agent of the present invention can also be used as a scaffold for wandering cells to bring about highly effective healing following surgery.

BRIEF DESCRIPTION OF THE DRAWINGS

FIG. 1 indicates a comparison of the mucosa-elevating effects of a 1% aqueous peptide solution, 0.5% aqueous peptide solution, 0.25% aqueous peptide solution and MucoUp in rabbit gastric mucosa (before injection).

FIG. 2 indicates a comparison of the mucosa-elevating effects of a 1% aqueous peptide solution, 0.5% aqueous peptide solution, 0.25% aqueous peptide solution and MucoUp in rabbit gastric mucosa (immediately after injection) ((a) 1% aqueous peptide solution, (b) 0.5% aqueous peptide solution, (c) 0.25% aqueous peptide solution, (d) 0.1% aqueous peptide solution, (e) MucoUp).

FIG. 3 indicates a comparison of the mucosa-elevating effects of a 1% aqueous peptide solution, 0.5% aqueous peptide solution, 0.25% aqueous peptide solution and MucoUp in rabbit gastric mucosa (after 15 minutes) ((a) 1% aqueous peptide solution, (b) 0.5% aqueous peptide solution, (c) 0.25% aqueous peptide solution, (d) 0.1% aqueous peptide solution, (e) MucoUp).

FIG. 4 indicates the results of submucosal layer dissection using a 0.25% aqueous peptide solution in miniature pig gastric mucosa ((a) before initial injection, (b) after initial injection, (c) during dissection, (d) after additional injection, (e) following completion of dissection).

FIG. 5 indicates the results of observing a pathological section of a dissected specimen following dissection of the submucosal layer of miniature pig gastric mucosa ((a) ○×○, (b) oxo).

In addition, since the main component of the mucosa-elevating agent of the present invention in the form of a self-assembling peptide can be produced synthetically, in addition to being able to eliminate the risk of viral and other infectious diseases in comparison with conventional biological materials, since the self-assembling peptide per se is bioabsorbable, there is no need for concern over inflammation and the like.

BEST MODE FOR CARRYING OUT THE INVENTION

The following provides a detailed explanation of the mucosa-elevating agent of the present invention.

The mucosa-elevating agent of the present invention has for the main component thereof an amphipathic peptide having 8 to 200 amino acid residues in which hydrophilic amino acids and hydrophobic amino acids are alternately bonded, and is a self-assembling peptide that exhibits a β structure in an aqueous solution at physiological pH and/or in the presence of cations.

In the present invention, physiological pH refers to pH 6 to pH 8, preferably pH 6.5 to pH 7.5 and more preferably pH 7.3 to pH 7.5. In addition, cations in the present invention refer to, for example, 5 mM to 5 M sodium ions or potassium ions.

The self-assembling peptide used in the present invention can be represented with, for example, the following four general formulas:

$$((XY)_l\text{-}(ZY)_m)_n \qquad (I)$$

$$((YX)_l\text{-}(YZ)_m)_n \qquad (II)$$

$$((ZY)_l\text{-}(XY)_m)_n \qquad (III)$$

$$((YZ)_l\text{-}(YX)_m)_n \qquad (IV)$$

(wherein, X represents an acidic amino acid, Y represents a hydrophobic amino acid, Z represents a basic amino acid, and l, m and n all represent integers (n x (l + m) < 200).

In addition, the N-terminal thereof may be acetylated and the C-terminal may be amidated.

Here, acidic amino acids selected from among aspartic acid and glutamic acid, and basic amino acids selected from among arginine, lysine, histidine and ornithine, can be used as hydrophilic amino acids. Alanine, valine, leucine, isoleucine, methionine, phenylalanine, tyrosine, tryptophan, serine, threonine and glycine can be used as hydrophobic amino acids.

Among these self-assembling peptides, self-assembling peptides having a repetitive sequence consisting of arginine, alanine, aspartic acid and alanine (RADA) can be used preferably, and this peptide sequence is represented by $Ac\text{-}(RADA)_p\text{-}CONH_2$ (p = 2 to 50). In addition, a self-assembling peptide having a repetitive sequence consisting of isoleucine, glutamic acid, isoleucine and lysine (IEIK) can also be used preferably, and this peptide sequence is represented by $Ac\text{-}(IEIK)_p I\text{-}CONH_2$ (p = 2 to 50). Moreover, a self-assembling peptide having a repetitive sequence consisting of leucine, aspartic acid and leucine (KLDL) can also be used preferably, and this peptide sequence is represented by $Ac\text{-}(KLDL)_p\text{-}CONH_2$ (p = 2 to 50). Although these self-assembling peptides can be consisted of 8 to 200 amino acid residues, self-assembling peptides having 8 to 32 residues are preferable, while self-assembling peptides having 12 to 16 residues are more preferable.

Specific examples of preferable self-assembling peptides in the present invention include peptide RAD16-I having the sequence ($Ac\text{-}RADA)_4\text{-}CONH_2$) (SEQ ID NO: 1), peptide IEIK13 having the sequence ($Ac\text{-}(IEIK)_3\text{-}CONH_2$) (SEQ ID NO: 2), and peptide KLD having the sequence ($Ac\text{-}KLDL)_3\text{-}CONH_2$ (SEQ ID NO: 3), and RAD16-I is commercially available under the trade name PuraMatrix® from 3D Matrix Inc. in the form of a 1% aqueous solution. PuraMatrix® contains hydrogen ions and chloride ions in addition to 1% of a peptide having the sequence ($Ac(RADA)_4\text{-}CONH_2$) (SEQ ID NO: 1).

PuraMatrix®, IEIK13 and KLD are oligopeptides consisted of 12 to 16 amino acid residues and having a length of about 5 nm, and although a solution thereof is in the form of a liquid at an acidic pH, a self-assembling peptide forms when the pH is changed to a neutral pH resulting in the formation of nanofibers having a diameter of about 10 nm, and the peptide solution turns into a gel as a result thereof.

PuraMatrix® is an amphipathic peptide having an amino acid sequence in which hydrophilic amino acids in the form of positively charged arginine and negatively charged aspartic acid and a hydrophobic amino acid in the form of an alanine residue alternately repeat, IEIK13 is an amphipatic peptide having an amino acid sequence in which hydrophilic amino acids in the form of positively charged lysine and negatively charged glutamic acid and a hydrophobic amino acid in the form of an isoleucine reside alternately repeat, and KLD is an amphipathic peptide having an amino acid sequence in which hydrophilic amino acids in the form of positively charged lysine and negatively charged aspartic acid and a hydrophobic amino acid in the form of a lysine residue alternately repeat, and peptide self-assembly is the result of hydrogen bonding and hydrophobic bonding between peptide molecules by amino acids that compose the peptide.

The average diameter of nanofibers in the self-assembling peptide used in the present invention is 10 nm to 20 nm and the pore size is 5 nm to 200 nm. As a result of being within these ranges, the self-assembling peptide of the present invention is of nearly the same size as collagen, which is a naturally-occurring extracellular matrix.

Examples of conditions for self-assembly of the self-assembling peptide used in the present invention include the physiological conditions of pH and salt concentration. The presence of monovalent alkaline metal ions is particularly important. In other words, sodium ions and potassium ions present in large amounts in the body contribute to promotion of gelling. Once gelled, the gel does not decompose even under conditions that cause denaturation of ordinary proteins, such as subjecting to high temperature, acid, base or protease, or subjecting to a denaturing agent such as urea or guanidine hydrochloride.

Since PuraMatrix® and these other self-assembling peptides having a peptide sequence that does not have a clearly defined physiologically active motif, there is no concern over the loss of inherent cell function. Physiologically active motifs are involved in the control of transcription and numerous other intracellular phenomena, and when a physiologically active motif is present, proteins within the cytoplasm or on the cell surface are phosphorylated by an enzyme that recognizes that motif. If a physiologically active motif is present in a peptide mucosa-elevating agent, there is the possibility of the transcription activities of various types of functional proteins being activated or inhibited. A self-assembling peptide such as PuraMatrix® does not present such concerns since it does not have a physiologically active motif.

Since the self-assembling peptide used in the present invention is produced by chemical synthesis, it does not contain unknown components arising from an animal-derived extracellular matrix. This property indicates that the self-assembling peptide is free of the risk of BSE and other infections and has a high degree of safety even if used in medical applications.

Since self-assembling peptides consisted of naturally-occurring amino acids have favorable biocompatibility and biodegradability, it has been reported that when PuraMatrix® was injected into mouse cardiac muscle, for example, cells infiltrated the injected PuraMatrix® resulting in the formation of normal tissue. Although decomposition time varies according to conditions such as the injection site, fibers are decomposed and excreted in about 2 to 8 weeks after injection.

The mucosa-elevating agent of the present invention makes it possible to further enhance biosafety by improving the osmotic pressure of a solution from hypotonicity to isotonicity without causing a decrease in mucosa-elevating effects by adding a sugar.

Examples of the form of the mucosa-elevating agent of the present invention include a powder, solution and gel. Since the self-assembling peptide turns into a gel due to a change in the pH or salt concentration of a solution, it can be distributed in the form of a liquid preparation that turns into a gel when contacted with the body at the time of application.

The mode of the mucosa-elevating agent during clinical use employs a method such as preliminarily filling a liquid preparation containing components such as the self-assembling peptide into a syringe cylinder or pipette (such as in the form of a pre-filled syringe), or supplying the liquid preparation to a syringe or pipette tip by a means (aspirator or valve) for replenishing components from the opening of a syringe or pipette tip and applying to an affected area from the portion from which the liquid preparation is released. The mode of use may also be consisted of two or more syringes or pipettes.

Although the following provides a more detailed explanation of the mucosa-elevating agent of the present invention through examples thereof, the present invention is not limited thereto provided there is no deviation from the gist and scope of application thereof.

EXAMPLE 1

Elevating effects on gastric submucosal layer of gastric submucosal injection in rabbits

Mucosa-elevating effects were evaluated by injection of a 0.25% aqueous peptide solution, 0.5% aqueous peptide solution, 1.0% aqueous peptide solution or MucoUp (Seikagaku Corp.) into the gastric submucosal layer of live rabbits.

<Materials>

Aqueous Peptide Solutions:

1. 0.25% aqueous peptide solution (peptide sequence: Ac-(RADA)$_4$-NH$_2$, CPC Scientific, Inc., concentration: weight/volume)
2. 0.5% aqueous peptide solution (peptide sequence: Ac-(RADA)$_4$-NH$_2$, CPC Scientific, Inc., concentration: weight/volume)
3. 1.0% aqueous peptide solution (peptide sequence: Ac-(RADA)$_4$-NH$_2$, CPC Scientific, Inc., concentration: weight/volume)
4. MucoUp (Seikagaku Corp., approval no.: 21800BZZ1012400)

Animals:

Japanese white rabbits (3.0 kg to 4.0 kg, Japan White, conventional, purchased from Funabasi Farm Co.) were used. The animals were housed in an animal breeding room controlled to a room temperature of 25°C, humidity of 65% and lighting time of 12 hours (7:00 to 19:00), fed laboratory animal feed pellets (JA Higashi Nihon Kumiai Shiryo Co., Ltd.) and given free access to drinking water from a water bottle. The animals were fasted only on the morning of the day of testing but were continued to be given free access to drinking water.

<Methods>

The rabbits were anesthetized by intravenous administration (10 mg/kg) of Ketamine (containing 50 mg as ketamine per ml, Fuji Chemical Industries, Ltd.) following subcutaneous administration (3 mg/kg) of 2% Secratal injection preparation (containing 2.0 g as xylazine in 100 mL, Bayer Inc.).

- The rabbits were laparotomized by median incision. An incision was made in the body of the stomach with a scalpel to expose the gastric mucosa.
- 0.5 mL of aqueous peptide solution were injected into the gastric submucosal layer with a 23G syringe (Terumo Corp.).

The height of mucosal elevation was measured macroscopically with a caliper immediately after and 15 minutes after injection of the aqueous peptide solutions or MucoUp.

<Results>

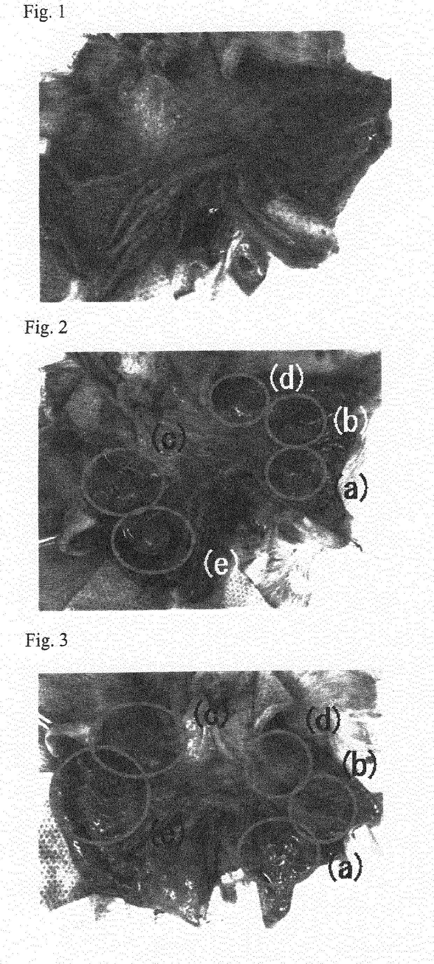

Examples of the mucosa-elevating effects of the aqueous peptide solutions of the present example or MucoUp are shown in Table 1 before administration (FIG. 1), immediately after administration (FIG. 2), 15 minutes after administration (FIG. 3) and 30 minutes after administration into the rabbit gastric submucosal layer. The solutions were evaluated as being effective as mucosa-elevating materials in the case the height of mucosal elevation 15 minutes after administration was maintained at 50% or more. The 1% aqueous peptide solution, 0.5% aqueous peptide solution, 0.25% aqueous peptide solution and MucoUp were observed to demonstrate effects that maintain the height of mucosal elevation.

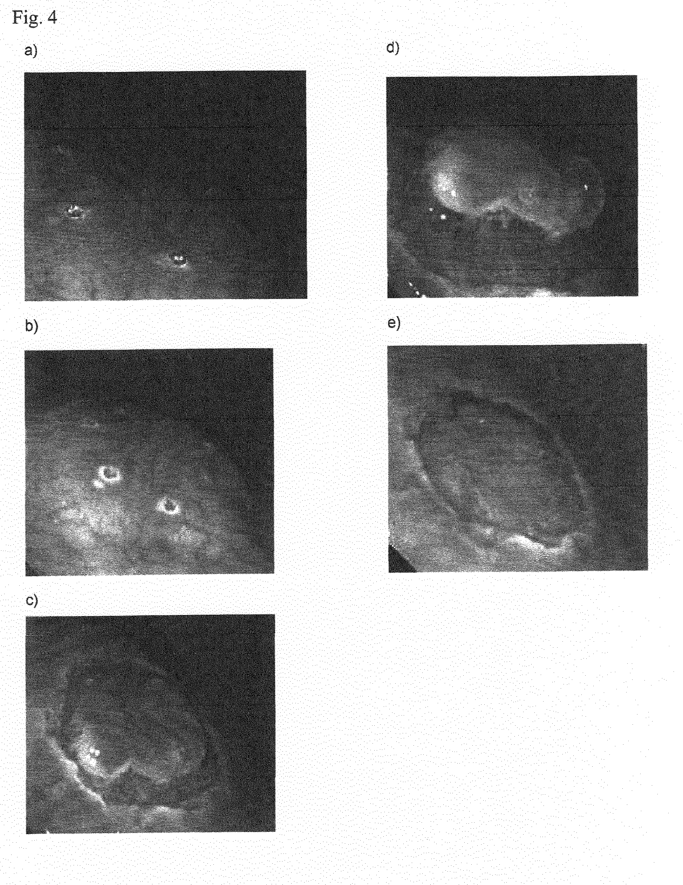

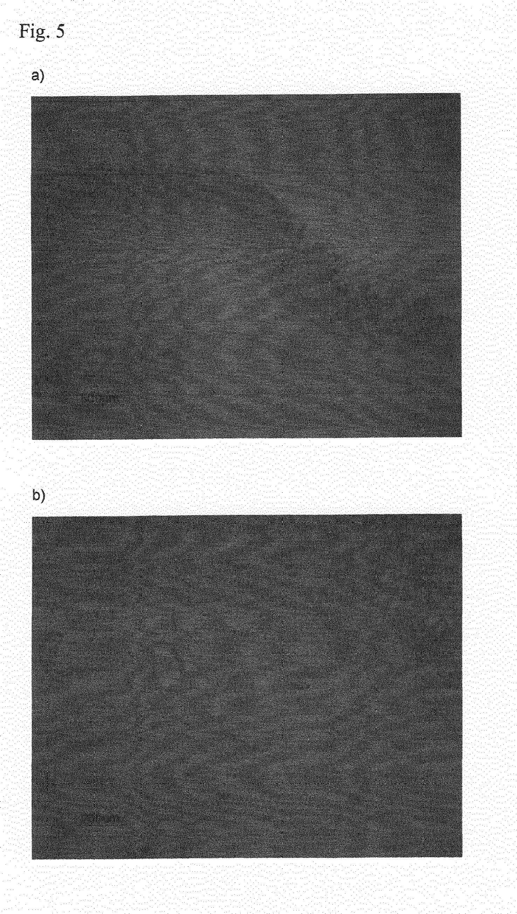

In addition, an example of evaluating the elevating effects on abdominal skin before administration (FIG. 4), immediately after administration (FIG. 5), 15 minutes after administration and 30 minutes after administration (FIG. 6) of aqueous peptide solutions of the present embodiment, MucoUp or physiological saline beneath the skin of the abdomen in rabbits for reference purposes are shown in Table 2. The aqueous peptide solutions were confirmed to have elevating effects equal to those of MucoUp 15 minutes and 30 minutes after administration, and the physical strength of the aqueous peptide solutions was also confirmed to be equal to that of MucoUp.

[Table 1]

| Elevating effects on gastric submucosal layer in rabbits | | | | | |
|---|---|---|---|---|---|
| | Evaluated Individual A | | | Evaluated Individual B | |
| | Immediately after | 15 minutes after | 30 minutes after | Immediately after | 15 minutes after |
| 1% aqueous peptide solution | 5 mm | 4 mm | 4 mm | 4 mm | 3 mm |
| 0.5% aqueous peptide solution | 4 mm | 3 mm | 3 mm | 3 mm | 2 mm |
| 0.25% aqueous peptide solution | 4 mm | 3 mm | 3 mm | 2 mm | 1 mm |
| 0.1% aqueous peptide solution | 2 mm | 1 mm | 1 mm | 2 mm | 1 mm |
| MucoUp | 4 mm | 3 mm | 3 mm | 4 mm | 3 mm |

[Table 2]

| | Evaluated Individual A | | | Evaluated Individual B | |
|---|---|---|---|---|---|
| Gastric subcutaneous elevating effects in rabbits | | | | | |
| | Immediately after | 15 minutes after | 30 minutes after | Immediately after | 15 minutes after |
| 1% aqueous peptide solution | 6 mm | 4 mm | 3 mm | 5 mm | 4 mm |
| 0.5% aqueous peptide solution | 4 mm | 3 mm | 2 mm | 5 mm | 3 mm |
| 0.25% aqueous peptide solution | 1 mm | 1 mm | 0 mm | 3 mm | 3 mm |
| 0.1% aqueous peptide solution | 5 mm | 4 mm | 1 mm | 4 mm | 2 mm |
| MucoUp | 4 mm | 3 mm | 1 mm | 4 mm | 1.5 mm |
| Physiological saline | 1 mm | 0 mm | 0 mm | 2 mm | 0 mm |

EXAMPLE 2

Elevating effects on gastric submucosal layer of gastric submucosal injection in miniature pigs

Elevating effects on the gastric submucosal layer during endoscopic gastric submucosal layer dissection in miniature pigs were evaluated for a 0.25% aqueous peptide solution.

<Materials>

Aqueous Peptide Solution:

1. 0.25% aqueous peptide solution (peptide sequence: Ac-(RADA)$_4$-NH$_2$, CPC Scientific, Inc.)

Animals:

NIBS miniature pigs age 20 to 21 months and weighing 20 kg to 40 kg (Nisseiken Co., Ltd.)

Measurement of body weight (electronic balance: DUE600ST/ID3S-A, Mettler-Toledo International Corp.), visual examinations and palpation examinations were performed on all animals at time of acquisition, and those animals observed to be free of abnormalities were placed in an animal breeding room. Subsequently, following a seven-day quarantine period, an 11-day acclimation period was further provided. During that time, the animals were quarantined and acclimated while measuring body weights four times (electronic balance: DUE600ST/ID3S-A, Mettler-Toledo International Corp.), measuring food consumption once per day (electronic balance: using either the PB1501 or PB3002-S/FACT, Mettler-Toledo International Corp.) and observing general condition once per day. The animals were housed in an animal breeding room maintained at a set temperature of 23°C (allowable range: 20°C to 28°C), relative humidity of 55% (allowable range: 30% to 80%), light-dark cycle of 12 hours each (light: 6:00 AM to 6:00 PM), and ventilation rate of 10 times/hour (by circulating fresh air through a filter). The animals were individually housed using stainless steel cages (W: 590 x D: 840 x H: 740 mm or W: 630 x D: 1130 x H: 710 mm) during the quarantine/acclimation period and measurement period. Solid feed (NS, Nisseiken Co., Ltd.) within five months of manufacturing was given in the morning at the rate of 500 g ± 5 g per day (321 kcal per 100 g, 1605 kcal per day, electronic balance: using either the PB3002-S/FACT or PB1501). However, the animals were fed an enteral nutrient preparation (Elental, Ajinomoto Pharma Co., Ltd.) having the same number of calories (fed amount: 628 g ± 10 g) 3 days prior and 2 days prior to surgery (counting the day of surgery as day 0) in order to facilitate the operability of endoscopic submucosal layer dissection, and feeding was discontinued starting on the day before surgery. Analysis of the same lot of solid feed as the solid feed used (NS) was carried out by acquiring data from testing carried out at Nisseiken Co., Ltd. and Seikan Co., Ltd. and confirming that analysis results were within the range of standard values determined by the testing facilities. Tap water was used for drinking water and the animals were given free access through the use of an automatic water dispenser. Testing of the drinking water quality was carried out by acquiring data from testing carried out at Toyo Kensa Center Co., Ltd.

roughly every six months and confirming that test results were within the range of standard values determined by the testing facility.

<Methods>

The miniature pigs were anesthetized by induction of anesthesia by intramuscular administration into muscle of the back of 0.05 mg/kg of atropine sulfate and 15 mg/kg of ketamine hydrochloride and maintaining anesthesia under conditions of a mixed gas of $N_2O$ and $O_2$ at a ratio of 1:1 and 0.5% to 1.5% isoflurane using an inhalation anesthetizer (Safer100, Anes Co., Ltd.). A catheter was inserted into the cranial vena cava (MediCut LCV-UK Kit, Nippon Sherwood Medical Industries, Ltd., barrel filled with heparinized saline (approx. 10 units/mL)) and immobilized by suturing to the neck. The catheter was wrapped around the back, the entirety was affixed to the body with an adhesive stretchable cloth bandage, and further protected with an elastic mesh bandage.

The endoscopic instrument (Olympus Corp.) was inserted orally into the stomach.
A sprinkling tube (Olympus Corp.) was inserted through the forceps port.
5 mL of administered specimen in the form of the aqueous peptide solution were locally injected into the gastric submucosal layer to elevate the surface of the mucosa followed by dissecting the elevated submucosal layer using an electric scalpel.
5 mL of peptide solution were additionally injected locally during dissection of the submucosal layer.
Following completion of dissection, the resected mucosal tissue was fixed with formalin and subjected to histopathological examination by hematoxylin and eosin staining.

<Results>

FIG. 4 indicates an example of the results of evaluating mucosa-elevating effects of an aqueous peptide solution during endoscopic submucosal layer dissection of the present example. As shown in Table 3, mucosa-elevating effects were confirmed to be sufficient for carrying out gastric submucosal layer dissection with a 0.25% aqueous peptide solution. In addition, as a result of observing a histopathological section of the resected gastric mucosa specimen, the aqueous peptide solution was confirmed to have been injected between the individual muscle fascia and mucous membrane, and the submucosal layer was confirmed to have been dissected (FIG. 5).

[Table 3]

| Elapsed time from initial injection to additional injection | Elapsed time from additional injection to completion of dissection | Findings |
| --- | --- | --- |
| 5 minutes 17 seconds | 4 minutes 40 seconds | No effect on dissection effects of high-frequency scalpel |

## Claims

1. A mucosa-elevating agent containing 0.1 % to 1.0% of a peptide, wherein the peptide is an amphipathic protein having 8 to 200 amino acid residues in which hydrophilic amino acids and hydrophobic amino acids are alternately bonded, and is a self-assembling peptide that exhibits a β structure in an aqueous solution at physiological pH and/or in the presence of cations.

2. The mucosa-elevating agent according to claim 1, wherein the peptide has a repetitive sequence of a sequence consisting of arginine, alanine, aspartic acid and alanine, a sequence consisting of isoleucine, glutamic acid, isoleucine and lysine, or a sequence consisting of lysine, leucine, aspartic acid and leucine.

3. The mucosa-elevating agent according to claim 1 or 2, wherein the peptide is consisted of the amino acid sequence described in SEQ ID NO: 1, SEQ ID NO: 2 or SEQ ID NO: 3.

4. The mucosa-elevating agent according to any of claims 1 to 3, further containing a pharmaceutical agent.

5. The mucosa-elevating agent according to claim 4, wherein the pharmaceutical agent is a pharmaceutically acceptable pigment.

6. The mucosa-elevating agent according to claim 4, wherein the pharmaceutical agent is selected from the group consisting of glucose, sucrose, refined sucrose, lactose, maltose, trehalose, dextran, iodine, lysozyme chloride, dimethyl isopropylazulene, tretinoin tocoferil, iodopovidone, alprostadil alfadex, anisyl alcohol, isoamyl salicylate, α,α-dimethylphenylethyl alcohol, bacdanol, helional, silver sulfadiazine, bucladesine sodium, alprostadil alfadex, gentamycin sulfate, tetracycline hydrochloride, fusidate sodium, mupirocin calcium hydrate and isoamyl benzoate.

7. The mucosa-elevating agent according to any of claims 1 to 6, which is injected between a mucous membrane and a muscle layer.

8. The mucosa-elevating agent according to any of claims 1 to 6, wherein the mucous membrane is gastrointestinal mucosa.

9. The mucosa-elevating agent according to any of claims 1 to 6, which is used in mucosal resection.

10. The mucosa-elevating agent according to any of claims 1 to 6, which is used in submucosal layer dissection.

11. The mucosa-elevating agent according to any of claims 1 to 6, which can be additionally injected.

12. The mucosa-elevating agent according to any of claims 1 to 6, which is in a form of being filled in a syringe.

13. The mucosa-elevating agent according to any of claims 1 to 6, which has a wound-healing effect.

**14.** The mucosa-elevating agent according to any of claims 1 to 6, which has a scar-preventing effect or constriction-preventing effect.

**15.** The mucosa-elevating agent according to any of claims 1 to 6, which has a hemostatic effect.

**16.** The mucosa-elevating agent according to any of claims 1 to 6, which is in a liquid form that gels in the body.

Fig. 1

Fig. 2

Fig. 3

Fig. 4

a)

b)

c)

d)

e)

Fig. 5

a)

b)

## INTERNATIONAL SEARCH REPORT

| International application No. |
| --- |
| PCT/JP2013/056472 |

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
A61L31/00, A61K9/06, A61K9/08, A61K38/00, A61K47/14, A61K47/22, A61K47/26, A61K47/36, A61K47/42, A61P7/04, A61P17/02, A61P41/00, A61P43/00

Documentation searched other than minimum documentation to the extent that such documents are included in the fields searched
Jitsuyo Shinan Koho           1922-1996    Jitsuyo Shinan Toroku Koho    1996-2013
Kokai Jitsuyo Shinan Koho     1971-2013    Toroku Jitsuyo Shinan Koho    1994-2013

Electronic data base consulted during the international search (name of data base and, where practicable, search terms used)
CA/MEDLINE/EMBASE/BIOSIS(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2010/41636 A1 (3-D Matrix, Ltd.), 15 April 2010 (15.04.2010), claims; paragraphs [0033] to [0048]; example 7 & US 2011/0201541 A1   & EP 2345433 A1 & CN 102170919 A     & KR 10-2011-0083648 A & RU 2011118341 A | 1-16 |
| Y | WO 2002/56914 A1 (Hironori YAMAMOTO), 25 July 2002 (25.07.2002), claims; page 2, line 6 to page 4, line 27; examples & JP 4176475 B          & US 2004/0059066 A1 & EP 1352661 A1 | 1-16 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 May, 2013 (01.05.13) | 14 May, 2013 (14.05.13) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

| International application No. |
|---|
| PCT/JP2013/056472 |

C (Continuation).　DOCUMENTS CONSIDERED TO BE RELEVANT

| Category* | Citation of document, with indication, where appropriate, of the relevant passages | Relevant to claim No. |
|---|---|---|
| Y | JP 2007-14799 A  (Seikagaku Corp.),<br>25 January 2007 (25.01.2007),<br>claims; paragraphs [0004] to [0022]; examples<br>& US 2003/0105061 A1　　& EP 1308164 A2 | 1-16 |
| Y | JP 2007-130118 A  (National Institute of<br>Advanced Industrial Science and Technology),<br>31 May 2007 (31.05.2007),<br>claims; examples<br>(Family: none) | 1-16 |
| Y | JP 2007-105186 A  (Nagoya University),<br>26 April 2007 (26.04.2007),<br>claims; paragraph [0021]; examples<br>(Family: none) | 1-16 |
| Y | WO 2010/103887 A1  (Menicon Co., Ltd.),<br>16 September 2010 (16.09.2010),<br>claims; examples<br>& US 2012/0058066 A1　　& EP 2407479 A1<br>& CN 102348717 A　　　　& TW 201036990 A<br>& KR 10-2011-0134440 A | 1-16 |
| A | JP 2009-90031 A  (National University<br>Corporation Chiba University),<br>30 April 2009 (30.04.2009),<br>claims; paragraph [0012]; examples<br>(Family: none) | 1-16 |

Form PCT/ISA/210 (continuation of second sheet) (July 2009)

**INTERNATIONAL SEARCH REPORT**

International application No.

PCT/JP2013/056472

Continuation of A. CLASSIFICATION OF SUBJECT MATTER
  (International Patent Classification (IPC))

A61L31/00(2006.01)i, A61K9/06(2006.01)i, A61K9/08(2006.01)i,
A61K38/00(2006.01)i, A61K47/14(2006.01)i, A61K47/22(2006.01)i,
A61K47/26(2006.01)i, A61K47/36(2006.01)i, A61K47/42(2006.01)i,
A61P7/04(2006.01)i, A61P17/02(2006.01)i, A61P41/00(2006.01)i,
A61P43/00(2006.01)i

        (According to International Patent Classification (IPC) or to both national
        classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010041636 A

SEQUENCE LISTING

<110>   3-D Matrix, Ltd.
<120>   Mucosa-raising agent
<130>   FP-3549PCT
<160>   4
<170>   PatentIn version 3.1

<210>   1
<211>   16
<212>   PRT
<213>   Artificial

<220>
<223>   designed peptide

<220>
<221>   MOD_RES
<222>   (1)..(1)
<223>   ACETYLATION

<400>   1

Arg Ala Asp Ala Arg Ala Asp Ala Arg Ala Asp Ala Arg Ala Asp Ala
1               5                   10                  15

<210>   2
<211>   13
<212>   PRT
<213>   Artificial

<220>
<223>   designed peptide

<220>
<221>   MOD_RES
<222>   (1)..(1)
<223>   ACETYLATION

<400>   2

Ile Glu Ile Lys Ile Glu Ile Lys Ile Glu Ile Lys Ile
1               5                   10

<210>   3
<211>   12
<212>   PRT
<213>   Artificial

<220>
<223>   desigend peptide

<220>
<221>   MOD_RES
<222>   (1)..(1)
<223>   ACETYLATION

<400>   3

Lys Leu Asp Leu Lys Leu Asp Leu Lys Leu Asp Leu
1               5                   10

<210>   4
<211>   9

```
<212>   PRT
<213>   Artificial

<220>
<223>   designed peptide

<220>
<221>   MOD_RES
<222>   (1)..(1)
<223>   ACETYLATION

<400>   4

Ile Glu Ile Lys Ile Glu Ile Lys Ile
1               5
```